# EUROPEAN PATENT SPECIFICATION

(11) **EP 2 659 892 B1**
(45) Date of publication and mention of the grant of the patent: **23.05.2018**
(21) Application number: 11853728.1
(22) Date of filing: 15.12.2011
(51) Int. Cl.: A61K 31/685, A61P 25/00, A61P 25/16, A61P 25/28, A61P 43/00

(54) **CONCOMITANT DRUG FOR IMPROVING COGNITIVE FUNCTION**
KONKOMITANTER WIRKSTOFF ZUR VERBESSERUNG DER KOGNITIVEN FUNKTION
MÉDICAMENT CONCOMITANT DESTINÉ À AMÉLIORER LA FONCTION COGNITIVE

(30) Priority: 29.12.2010 JP 2010294487
(43) Date of publication of application: 06.11.2013
(73) Proprietor: Nishizaki Bioinformation Research Institute, Kobe-shi, Hyogo 651-1223 (JP)
(72) Inventor: NISHIZAKI, Tomoyuki, Kobe-shi Hyogo 651-1223 (JP)
(74) Representative: Gille Hrabal
(86) International application number: PCT/JP2011/078989
(87) International publication number: WO 2012/090713

(56) References cited:
- WO-A1-2005/027933
- JP-A- 10 084 880
- JP-A- 2009 007 329
- US-A1- 2006 008 517
- H.J. HEO ET AL: "Effects of Banana, Orange, and Apple on Oxidative Stress-Induced Neurotoxicity in PC12 Cells", JOURNAL OF FOOD SCIENCE, vol. 73, no. 2, 1 March 2008 (2008-03-01), pages H28-H32, XP055174920, ISSN: 0022-1147, DOI: 10.1111/j.1750-3841.2007.00632.x
- Michael Murray: "The Encyclopedia of Healing Foods", 1 January 2005 (2005-01-01), XP002737012, page 382, * page 382, column 1, paragraph 4 *
- MICHIYUKI KOJIMA ET AL: "Structural Relationships of 1, 2-Diacylglycerol Residues in Glycerolipid Classes from Seeds, Leaves and Roots of Adzuki bean (Vigna angularis)", JOURNAL OF JAPAN OIL CHEMISTS' SOCIETY, vol. 38, no. 8, 1 January 1989 (1989-01-01), pages 619-625, XP055174990, DOI: 10.5650/jos1956.38.619
- OSTATNIKOVA ET AL: "Short-term soybean intake and its effect on steroid sex hormones and cognitive abilities", FERTILITY AND STERILITY, ELSEVIER SCIENCE INC, NEW YORK, NY, USA, vol. 88, no. 6, 1 December 2007 (2007-12-01), pages 1632-1636, XP022413605, ISSN: 0015-0282, DOI: 10.1016/J.FERTNSTERT.2007.01.106
- NAGATA,T. ET AL.: 'DL- and PO- phosphatidylcholines as a promising learning and memory enhancer' LIPIDS HEALTH DIS vol. 10, no. 25, 28 January 2011, pages 1 - 5, XP021088505
- YOKO MATSUMOTO ET AL.: 'Comparative examination of the effects of egg yolk and soybean phosphatidylcholine on memories of the low memory mice' THE JAPANESE SOCIETY OF NUTRITION AND FOOD SCIENCE SOKAI KOEN YOSHISHU vol. 50TH, 1996, page 244, XP008168960
- XIA,H. ET AL.: 'Analysis of soybean phosphatidylcholine molecular species using high performance liquid chromatography' FENXI SHIYANSHI vol. 22, no. 1, 2003, pages 9 - 11, XP008168813

## Description

### Technical Field

The present invention relates to 1,2-dilinoleoyl-sn-glycero-3-phosphocholine (DLPC) and 1-palmitoyl-2-oleoyl-sn-glycero-3-phosphocholine (POPC) for combination use in a method of improving cognitive function.

### Background Art

In recent years, dementia has become a major medical problem worldwide. Dementia is a disease accompanied by various kinds of symptoms centering around learning and memory disorders and impaired judgment, and the symptoms and progress thereof vary depending on the diseases causing them. However, all cases are common in that the quality of life of patients is markedly impaired. Given the fact that caregivers including the patients' families are forced to offer a large amount of labor, dementia can be said a very serious problem at the social level. Since the increasing population of elderly citizens resulting from the increasing life span is related to the increase of dementia patients, the number of dementia patients is predicted to further increase in years ahead in Japan. In addition, there are many people suffering from any cognitive impairment which is not classified as dementia.

While many diseases have so far been indicated to cause dementia, cerebrovascular dementia and Alzheimer-type dementia are most popular, and the both and a composite type thereof occupy the majority of the causative diseases. Particularly, Alzheimer-type dementia has been increasing in Japan in recent years.

The detailed mechanism of the onset of dementia has not been clarified. However, various biochemical lesions have been reported in dementia patients. Reported in Alzheimer-type dementia, Lewy body dementia and the like is a decrease in the intracerebral acetylcholine concentration. Use of an acetylcholine degrading enzyme inhibitor based on this fact is the most successful method to the present for the treatment of dementia, particularly Alzheimer-type dementia. In Japan, various kinds of acetylcholine degrading enzyme inhibitors have heretofore been developed, including already commercially available donepezil hydrochloride (trade name Aricept). However, such medicaments do not fundamentally treat dementia but show an effect of delaying the progression of symptoms. As for donepezil hydrochloride, moreover, the problem of side effects such as the risk of developing acute renal failure, rhabdomyolysis and the like has been reported. For these reasons, the development of a drug for improving dementia, which is safer and shows high effect, has been desired, and such drug highly likely acts by a mechanism different from inhibition of acetylcholine degrading enzyme.

The present inventor has heretofore reported that, phosphatidylcholine as a medicament different from donepezil hydrochloride has a cognitive function improving effect (patent document 1). However, a medicament for improving the cognitive function is still desired as the situation stands.

### [Document List]

### [patent document]

patent document 1: JP-A-2009-7329

US 2006/008517 discloses a method for reducing symptoms associated with age related memory loss comprising administering to the subject an effective amount of phosphatidylgylcerol-carrying bodies.

WO 2005/027933 relates to unsaturated phosphatidylcholines and discloses in Table 2 on p. 57 that banana fruit, inside skin and yellow skin contain phosphatidylcholine species, and that lecithin contains DLCP and POPC.

H.J. Heo et al., Journal of Food Science, vol. 73, no. 2, 2008, p. H28-H32 discloses that that antioxidative agents, in particular phenolics, from bananas, orange and apple fruits might prevent oxidative-stress induced neurotoxicity.

M. Murray, "The Encyclopedia of Healing Foods", January 2005, p. 382 discloses that Adzuki beans contain, besides other trace minerals, molybdenum, which is important for sulfoxidation. Poor sulfoxidation, in turn, is associated with neurodegenerative diseases such as Alzheimer's or Parkinson's disease according to this textbook.

M. Kojima et al., Journal of Japan Oil Chemist's Society, vol. 38, no. 8, January 1989, p. 619-625 discloses that Adzuki beans contain DLPC and POPC, besides other glycerlipids. Information on ratios of various phospholipids found in different parts of the adzuki bean plant is provided.

Ostatnikova at al., Fertility and Sterility, Elsevier Science, vol. 88, no. 6, December 2007, p. 1632-1636 discloses that soy beans improve memory.

T. Nagata et al., Lipids Health DIS, vol. 10, no. 25, January 2011, p. 1-5 discloses a study assessing the efficacy of DLPhtCho, POPPhtCho, AA, DHA and PLPPhtSer on learning and memory functions. DL- and PO-phosphatidylcholines are considered a promising learning and memory enhancer. Y. Matsumoto et al., The Japanese Society of Nutrition and Food Science Sokai Koen Yoshishu, vol. 50TH, 1996, p. 244 discloses the use of soybean phosphatidyl choline to improve memory function.

H. Xia et al, Fenxi Shyanshi vol. 22, no. 1, 2003, p. 9-11 describes the analysis of soybean phosphatidylcholine molecular species using HPLC.

JP 10-084880 discloses a phospholipid-containing composition, preferably phosphatidyl choline or phosphatidyl ethanol amine, which has an effect to promote metabolism of lipid. JP 2009-007329 discloses a composition comprising various phosphatidylcholines having aliphatic acid residues for preventing or treating diseases or states involving gognitive impairment.

### SUMMARY OF THE INVENTION

### Problems to be Solved by the Invention

The present invention aims to provide a medicament for use in a method of improving cognitive function. Means of Solving the Problems

The present inventor has found from previous studies that phosphatidylcholine has a cognitive function improving effect. Based on such finding, therefore, the present inventor has conducted further intensive studies to obtain a medicament capable of improving the cognitive function more effectively. As a result, the present inventor has found that a combined use of particular two kinds of phosphatidylcholine is more effective for the improvement of the cognitive function, and further confirmed that it exhibits a sufficient effect in clinical practice, which resulted in the completion of the present invention. Accordingly, the present invention is as described below.
1. 1,2-dilinoleoyl-sn-glycero-3-phosphocholine (DLPC) and 1-palmitoyl-2-oleoyl-sn-glycero-3-phosphocholine (POPC) for combination use in a method of improving cognitive function.
2. DLPC and POPC for the use according to 1, wherein the improvement of cognitive function is that in a patient having a disease or condition associated with cognitive impairment.
3. DLPC and POPC for the use according to 2, wherein the disease or condition associated with cognitive impairment is at least one kind selected from the group consisting of dementia, non-dementia cognitive impairment and learning or memory disorders.

### Effect of the Invention

The DLCP and POPC for combination use of the present invention has a cognitive function improving effect and can be useful for the prophylaxis or treatment of, for example, various diseases or conditions including dementia, non-dementia cognitive impairment, learning or memory disorders and the like, or improvement of learning ability and/or memory ability.

### Brief Description of the Drawings

Fig. 1 is a graph showing the effects of administration of DLPC alone, administration of POPC alone, and combined administration of DLPC and POPC, on the acquisition latency of normal rats. Each point shows the mean (±SEM) of the acquisition latency for continuous 2 days. N=6
Fig. 2 is a graph showing the effects of combined administration of POPC and DLPC on cognitive impairment. Each value shows mean (±SEM) of MMSE score at each time point. ***P<0.0001, paired t-test.
Fig. 3 is a graph showing the effects of the administration of POPC alone, DLPC alone, or POPC+DLPC in combination on cognitive impairment. The difference in the MMSE scores before ingestion and 5 months after ingestion was calculated (Δ increase in MMSE score). Each column shows mean (±SEM) Δ increase in the MMSE score. P value, unpaired t-test. Description of Embodiments

The present invention is explained in detail in the following.

The present invention is characterized by two kinds of particular phosphatidylcholines for combination use in a method for improving cognitive function.

One of the phosphatidylcholines is dilinoleoyl phosphatidylcholine represented by the following formula 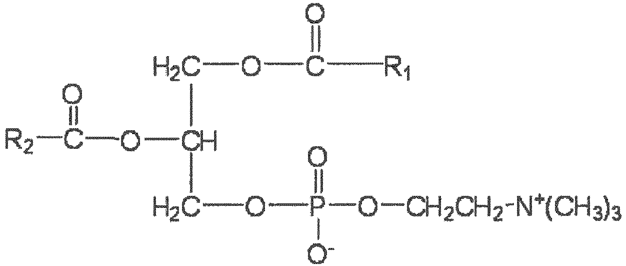 wherein -C(O)R₁ and -C(O)R₂ are each a linoleic acid residue, (1,2-dilinoleoyl-sn-glycero-3-phosphocholine; hereinafter DLPC).

The other phosphatidylcholine is palmitoyloleoyl phosphatidylcholine represented by the following formula 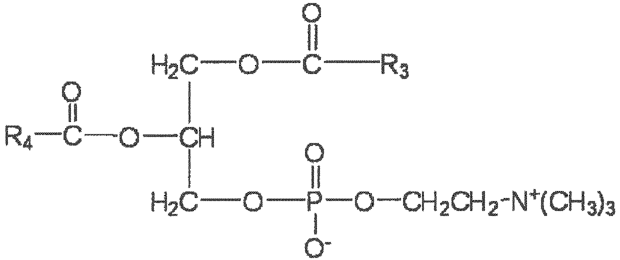 wherein -C(O)R₃ is a palmitic acid residue, and -C(O)R₄ is an oleic acid residue, (1-palmitoyl-2-oleoyl-sn-glycero-3-phosphocholine; hereinafter POPC).

The DLPC and POPC may be derivatized in view of the activity and safety thereof. For example, derivatizations such as hydrogenation, hydroxylation, alkylation, halogenation and the like can be mentioned, but are not limited thereto.

The DLPC and POPC to be used in the present invention are not particularly limited and include those isolated and purified from animals (egg-yolk and the like), plants (soybean and the like), fungi (yeast, mold) and the like, those synthesized chemically and the like. In addition, the DLPC and POPC to be used in the present invention can be used without any particular limitation as long as they have been purified to the level permitting use as a medicament. Furthermore, a commercially available product can also be used.

The "concomitant drug" in the present disclosure means that the above-mentioned DLPC and POPC are used in combination for administration.

The administration form of the concomitant drug of the present disclosure is not particularly limited and DLPC and POPC only need to be combined at the time of administration. Examples of such administration form include
(1) administration as a single preparation obtained by simultaneously formulating DLPC and POPC,
(2) simultaneous administration of two kinds of preparations obtained by separately formulating DLPC and POPC, by the same administration route,
(3) administration of two kinds of preparations obtained by separately formulating DLPC and POPC, in a staggered manner by the same administration route,
(4) simultaneous administration of two kinds of preparations obtained by separately formulating DLPC and POPC, by different administration routes,
(5) administration of two kinds of preparations obtained by separately formulating DLPC and POPC, in a staggered manner by different administration routes,
and the like.

From the aspect of convenience, administration as a single preparation and simultaneous administration of two kinds of preparations by the same route are preferable.

In the following, the "preparation" in the present disclosure includes both a single preparation obtained by simultaneously formulating DLPC and POPC, and two kinds of preparations obtained by separately formulating DLPC and POPC.

The amount of each of DLPC and POPC in the concomitant drug of the present disclosure varies depending on the administration form of the concomitant drug (as mentioned above), severity of the disease, the animal species to be the subject of administration, drug acceptability, body weight and age of the subject of administration, and the like. Generally, 50 - 500 mg, preferably 100 - 300 mg, of DLPC and 50 - 500 mg, preferably 100 - 300 mg, of POPC are administered per day to an adult subject. The ingestion ratio of DLPC and POPC is preferably about 1:1. The dose of DLPC and POPC can be decreased when they are used in combination than when they are used singly.

The concomitant drug of the present disclosure can contain, besides DLPC and POPC as the active ingredients, any additive, for example, a pharmaceutically acceptable carrier. Examples of the pharmaceutically acceptable carrier include, but are not limited to, excipients such as sucrose, starch, mannit, sorbit, lactose, glucose, cellulose, talc, calcium phosphate, calcium carbonate and the like, binders such as cellulose, methylcellulose, hydroxypropylcellulose, polypropylpyrrolidone, gelatin, gum arabic, polyethylene glycol, sucrose, starch and the like, disintegrants such as starch, carboxymethylcellulose, hydroxypropylstarch, sodium-glycol-starch, sodium hydrogen carbonate, calcium phosphate, calcium citrate and the like, lubricants such as magnesium stearate, aerosil, talc, sodium lauryl sulfate and the like, aromatics such as citric acid, menthol, glycyllysin·ammonium salt, glycine, orange powder and the like, preservatives such as sodium benzoate, sodium bisulfite, methylparaben, propylparaben and the like, stabilizers such as citric acid, sodium citrate, acetic acid and the like, suspensions such as methylcellulose, polyvinylpyrrolidone, aluminum stearate and the like, dispersing agents such as surfactant and the like, diluents such as water, saline, orange juice and the like, base waxes such as cacao butter, polyethylene glycol, paraffin and the like, and the like.

In one embodiment, the concomitant drug of the present disclosure can be formulated as a preparation preferable for oral administration. The pharmaceutical preparation preferable for oral administration includes a liquid wherein an effective amount of a substance is dissolved in a diluent such as water and saline, a capsule, granule, powder or tablet, containing an effective amount of a substance as a solid or granule, a suspension wherein an effective amount of a substance is suspended in a suitable dispersing medium, an emulsion wherein a solution containing an effective amount of a substance dissolved therein is dispersed and emulsified in a suitable dispersing medium, and the like.

In another embodiment, the concomitant drug of the present disclosure can be formulated as a pharmaceutical preparation preferable for parenteral administration. The preparation preferable for parenteral administration (e.g., intravenous injection, subcutaneous injection, intramuscular injection, topical injection and the like) includes aqueous and non-aqueous isotonic sterile injection liquids, which may contain antioxidant, buffer, bacteriostatic agent, isotonicity agent and the like. Aqueous and non-aqueous sterile suspensions can also be mentioned, which may contain suspending agent, solubilizer, thickener, stabilizer, preservative and the like. Such preparation can be sealed in a container such as ampoule and vial by a unit dose or plural doses. In addition, it is also possible to freeze-dry the active ingredient and pharmaceutically acceptable carriers, and preserve them in a suitable sterile vehicle in a state only requiring dissolving or suspending immediately before use.

The concomitant drug of the present disclosure containing DLPC and POPC as the active ingredients has an action to improve cognitive function in mammals (e.g., mouse, rat, hamster, rabbit, cat, dog, bovine, sheep, monkey, human and the like). Therefore, the concomitant drug of the present disclosure containing DLPC and POPC is useful for the prophylaxis or treatment of diseases or conditions associated with cognitive impairment, and is provided as a pharmaceutical product. Specific examples of the diseases or conditions associated with cognitive impairment include various diseases and conditions including dementia (e.g., dementia caused by various diseases such as senile dementia, Alzheimer-type dementia, cerebrovascular dementia, posttraumatic dementia, dementia caused by brain tumor, dementia caused by chronic subdural hematoma, dementia caused by normal pressure hydrocephalus, dementia after meningitis, Parkinson-type dementia and the like), non-dementia cognitive impairment (e.g., mild cognitive impairment (MCI)), learning or memory disorders (e.g., learning and memory disorders associated with impaired brain development) and the like. Furthermore, the concomitant drug of the present disclosure can be used for the improvement of learning and memory (e.g., short term memory, long-term memory).

When used in the present specification, the "prophylaxis" means prevention of manifestation of cognitive impairment, learning or memory disorders and the like in a target (test subject) showing no such symptoms, and the "treatment" means reducing or preventing worsening of or delaying cognitive impairment, learning or memory disorders and the like in a test subject showing no such symptoms. The "improvement" means improvement of cognition ability and learning and memory ability in a target not showing cognitive impairment, learning or memory disorders and the like, and mitigation, preferably mitigation of the symptoms, of cognitive impairment, learning or memory disorders and the like to the level permitting normal daily life in a test subject showing such symptoms.

The concomitant drug of the present disclosure can be provided as a food. The concomitant drug of the present disclosure containing DLPC and POPC as active ingredients has an action to improve cognitive function in mammals (e.g., mouse, rat, hamster, rabbit, cat, dog, bovine, sheep, monkey, human and the like). Therefore, the concomitant drug of the present disclosure containing DLPC and POPC as active ingredients is effective for the prophylaxis or control of the diseases or conditions associated with cognitive impairment. Particularly, the concomitant drug can be provided as a functional food effective for the prophylaxis or control of the diseases or conditions associated with cognitive impairment, and as a functional food effective for the improvement of learning and memory.

The "food" in the present disclosure means any food and drink other than pharmaceutical products and quasi-drugs. For example, it includes, but is not limited to, foods for specified health uses, foods with nutrient function claims, and so-called supplements.

When the concomitant drug of the present disclosure is used as a food, the food includes, for example, general foods (e.g., bread, milk product (e.g., milk, yoghurt), confectionery, candy, drop, chocolate, cake, pudding, jelly, soft drink, noodles), health foods, dietary supplements, and foods for specified health uses and foods with nutrient function claims, which are defined in the food with health claims system by the Ministry of Health, Labour and Welfare. The foods can be in any form such as liquid (water-soluble, insoluble), solid such as powder, granule, tablet, capsule and the like, semi-sold such as jelly etc. and the like. The concomitant drug of the present disclosure can be used by dissolving in water or a predetermined aqueous solution. In this case, the concomitant drug of the present disclosure may contain a dissolution support (e.g., linoleic acid) and a stabilizer.

When the concomitant drug of the present disclosure is used as a food, the amount thereof to be ingested varies depending on the form of use (e.g., liquid, solid, semi-solid), concentration of DLPC and POPC contained, presence or absence, the kind and amount of a component to be contained besides DLPC and POPC, and the like, and cannot be generalized. Normally, DLPC and POPC are preferably contained in a food in a total amount of not less than 30%, more preferably not less than 90%. Examples of the components other than DLPC and POPC in the food include the above-mentioned optional additives.

The concomitant drug of the present disclosure may be in a form wherein a unit ingestion amount or a portion thereof is individually packed or filled, or the unit ingestion amount or a portion thereof in a large number are comprehensively packed or filled.

When the concomitant drug of the present disclosure is provided as a single preparation, the unit ingestion amount or a portion thereof is the unit ingestion amount or a portion thereof of the total amount of DLPC and POPC, that is, the total phosphatidylcholine. When the concomitant drug of the present disclosure is provided for a combined use of two kinds of preparations, the unit ingestion amount or a portion thereof of the concomitant drug is a combination of the unit ingestion amount or a portion thereof of DLPC, and the unit ingestion amount or a portion thereof of POPC.

Examples of the pharmaceutical product or a food wherein a unit ingestion amount or a portion thereof is individually packed or filled, those wherein a unit ingestion amount or a portion thereof is individually packed or filled in a general package (e.g., PTP (press through packing) sheet, paper container, film (e.g., plastic film) container, glass container, plastic container). Such pharmaceutical product or food individually packed or filled as above may be further combined and simultaneously packed or filled in one container (e.g., paper container, film (e.g., plastic film) container, glass container, plastic container). Examples of the pharmaceutical product or food wherein the unit ingestion amount or a portion thereof in a large number are comprehensively packed or filled include those wherein many tablets and capsules are packed or filled without sorting in one container (e.g., paper container, film (e.g., plastic film) container, glass container, plastic container). The pharmaceutical product or food of the present disclosure can also contain the unit ingestion amount or a portion thereof in a number sufficient for a long-term ingestion. In the case of a food, for example, in a number sufficient for 3 days or longer, preferably 7 days, 10 days, 14 days, 21 days or longer, or 1 month, 2 months, 3 months or longer.

The concomitant drug of the present disclosure may contain, in addition to DLPC and POPC as essential active ingredients, other one or more kinds of compounds capable of preventing or treating neurodegenerative diseases.

Examples of other compound for the prophylaxis or treatment of neurodegenerative diseases include polyphenol, coenzyme Q10, β-sitosterol, isoflavone, mevinic acid, vitamin C, vitamin E, flavonoids, terpenes, folic acid, vitamin B6, vitamin B12, sesquiterpene lactone, urokinase, nattokinase, dilinoleoyl phosphatidylethanolamine, propyl sulfide, apple pectin, acetic acid, EPA and DHA.

In the present invention, a combined administration of DLPC and POPC can improve cognitive function in mammals (e.g., mouse, rat, hamster, rabbit, cat, dog, bovine, sheep, monkey, human and the like). Therefore, administration of DLPC and POPC in combination can prevent or treat the diseases or conditions associated with cognitive impairment. Specific examples of the disease or condition associated with cognitive impairment include various diseases and conditions including dementia (e.g., dementia caused by various diseases such as senile dementia, Alzheimer-type dementia, cerebrovascular dementia, posttraumatic dementia, dementia caused by brain tumor, dementia caused by chronic subdural hematoma, dementia caused by normal pressure hydrocephalus, dementia after meningitis, and Parkinson-type dementia and the like), non-dementia cognitive impairment (e.g., mild cognitive impairment (MCI)), learning or memory disorders (e.g., learning and memory disorders associated with impaired brain development) and the like. Furthermore, administration of DLPC and POPC of the present invention in combination is expected to improve learning and memory (e.g., short term memory, long term memory). The dose and administration form of DLPC and POPC for concomitant use in the method of the present invention are the same as those mentioned for the above-mentioned concomitant drug of the present disclosure.

While the present invention is explained in more detail in the following by referring to Examples, the present invention is not limited by the following Examples and the like in any manner.

### Example

### (Experiment method and material)

### 1. Water maze test

Male Wister rats (7-weeks-old) were used for a water maze test. A circular plastic water tank (diameter 180 cm, depth 45 cm) was used. The inside of the water tank was completely painted in black, and dark water with India ink was filled up to 25 cm from the bottom (22°C). A platform (diameter 11 cm) painted in black was placed in water such that it was 1 cm below the water surface. The water tank was placed in a test room, and several marks seen by the rat from the water tank were put thereon. During the test, the position of the marks was not changed. A platform was placed a predetermined position from the equal distance from the center and the end of the water tank, namely, center of one quadrant. At one of the randomly selected 5 points, the rat was released facing the wall of the water tank, and the time necessary for evacuating on the platform (acquisition latency) was measured. When smoothly evacuated, the rat was left on the platform for 10 seconds. From 7 days before the water maze test and during the test, DLPC (5 mg/kg) alone, DLPC (10 mg/kg) alone, POPC (5 mg/kg) alone, POPC (10 mg/kg) alone, DLPC (5 mg/kg) and POPC (5 mg/kg), or PEG alone, each dissolved in polyethylene glycol (PEG), was orally administered every day. The water maze test was performed twice a day, and the second test was started at 2 min after the first test. The test was continuously performed for 8 days, and the mean (±SEM) of acquisition latency of continuous 2 days until the rat reached the platform was calculated.

### 2. Mini Mental State Examination (MMSE) test

An MMSE test was performed with 310 patients (135 males, 175 females, age 59 - 95, average age 76±1.1) having cognitive impairment. POPC (90 mg/day) was orally administered to 214 patients, DLPC (100 mg/day) to 21 patients, and DLPC (50 mg/day) and POPC (45 mg/day) to 75 patients, once after breakfast. In the MMSE test, the full score was 30, and less than 20 was evaluated as mild cognitive impairment and dementia.

### (Results)

### Experimental Example 1. Effect of DLPC single administration, POPC single administration, and combined administration of DLPC and POPC on acquisition latency of rat

PEG, DLPC (5 mg/kg), DLPC (10 mg/kg), POPC (5 mg/kg), POPC (10 mg/kg) or DLPC (5 mg/kg) and POPC (5 mg/kg) was orally administered to rats every day during the water maze test from 7 days before the test.

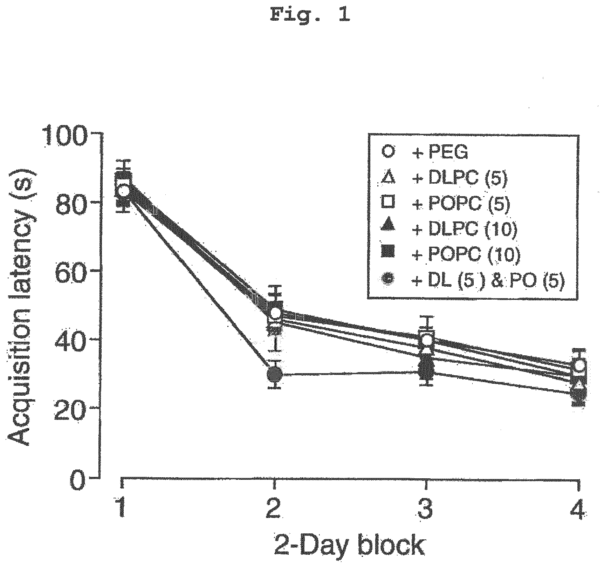

When DLPC (5 mg/kg) and POPC (5 mg/kg) were used in combination, the acquisition latency of the rat was remarkably shortened. In contrast, single administration of DLPC (5 mg/kg, 10 mg/kg) and single administration of POPC (5 mg/kg, 10 mg/kg) did not show a remarkable effect (Fig. 1).

This suggests that the combined use of DLPC and POPC can enhance learning and memory ability.

### Experimental Example 2. Effect of combined administration of POPC and DLPC on cognitive impairment

The effect of a combined administration of POPC and DLPC on cognitive impairment was examined by an MMSE test.

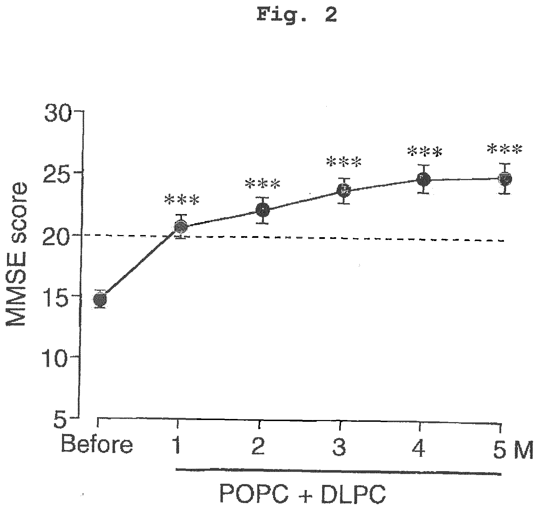

75 patients were orally ingested with POPC (50 mg/day) and DLPC (45 mg/day) once after breakfast, and the MMSE test was performed once per month. The results are shown in Fig. 2.

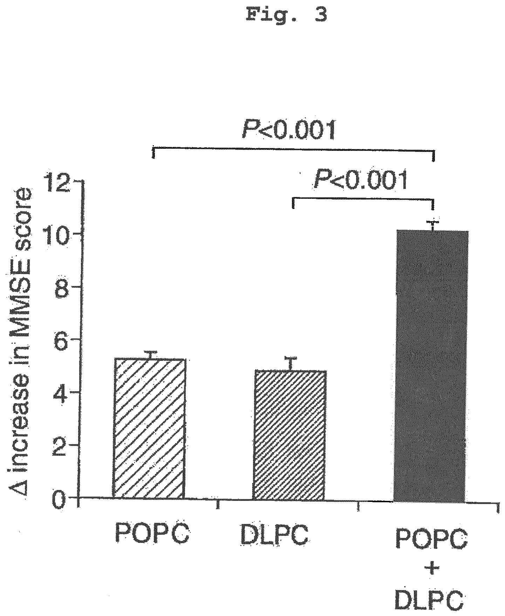

In the same manner, 214 patients orally ingested with POPC alone (90 mg/day, once after breakfast, every day), and 21 patients orally ingested with DLPC alone (100 mg/day, once after breakfast, every day) underwent the MMSE test once per month. The difference in the MMSE scores before and 5 months after the ingestion was calculated (Δ increase in MMSE score). The results are shown in Fig. 3.

About 65% of the 310 patients examined in the present test had mild cognitive impairment and dementia. The 75 patients ingested with both POPC and DLPC showed an average MMSE score before ingestion of 14.7±0.7 (Fig. 2). When DLPC (50 mg/day) and POPC (45 mg/day) were ingested in combination once after breakfast every day, the MMSE score remarkably increased, and the average score exceeded 20. That is, the patients recovered normal cognitive function 5 months after the ingestion (Fig. 2).

The patients after 5 months from the combined ingestion of DLPC (50 mg/day) and POPC (45 mg/day) showed a more remarkably-increased MMSE score as compared to the patients ingested with POPC (90 mg/day) alone, and the patients ingested with DLPC (100 mg/day) alone (Fig. 3).

These results show that a combined treatment of DLPC and POPC is more effective for the improvement of mild cognitive impairment and dementia, as compared to a treatment with each of POPC and DLPC alone.

### Industrial Applicability

The concomitant drug of the present disclosure has a cognitive function improving effect and can be useful for the prophylaxis or treatment of, for example, various diseases or conditions including dementia, non-dementia cognitive impairment, learning or memory disorders and the like, or improvement of learning ability and/or memory ability.

This application is based on a patent application No. 2010-294487 (filing date: December 29, 2010) filed in Japan.

## Claims

1. 1,2-dilinoleoyl-sn-glycero-3-phosphocholine (DLPC) and 1-palmitoyl-2-oleoyl-sn-glycero-3-phosphocholine (POPC) for combination use in a method of improving cognitive function.

2. DLPC and POPC for the use according to claim 1, wherein the improvement of cognitive function is that in a patient having a disease or condition associated with cognitive impairment.

3. DLPC and POPC for the use according to claim 2, wherein the disease or condition associated with cognitive impairment is at least one kind selected from the group consisting of dementia, non-dementia cognitive impairment and learning or memory disorders.

## Patentansprüche

1. 1,2-Dilinoleoyl-sn-glycero-3-phosphocholin (DLPC) und 1-Palmitoyl-2-oleoyl-sn-glycero-3-phosphocholin (POPC) zur kombinierten Verwendung in einem Verfahren zur Verbesserung der kognitiven Funktion.

2. DLPC und POPC für die Verwendung nach Anspruch 1, worin die Verbesserung der kognitiven Funktion diejenige eines Patienten ist, der eine Krankheit oder einen Zustand hat, der mit einer kognitiven Beeinträchtigung verbunden ist.

3. DLPC und POPC für die Verwendung nach Anspruch 1, worin die Krankheit oder der Zustand, die mit einer kognitiven Beeinträchtigung verbunden sind, mindestens einer Art ist, ausgewählt aus der Gruppe bestehend aus Demenz, kognitiver Beeinträchtigung ohne Demenz und Lern- oder Gedächtnisstörungen.

## Revendications

1. 1,2-Dilinoléoyl-sn-glycéro-3-phosphocholine (DLPC) et 1-palmitoyl-2-oléoyl-sn-glycéro-3-phosphocholine (POPC) pour leur utilisation en combinaison dans une méthode d'amélioration de la fonction cognitive.

2. DLPC et POPC pour leur utilisation selon la revendication 1, dans lesquelles l'amélioration de la fonction cognitive est celle chez un patient souffrant d'une maladie ou d'une affection associée à un trouble cognitif.

3. DLPC et POPC pour leur utilisation selon la revendication 2, dans lesquelles la maladie ou l'affection associée au trouble cognitif est au moins un type sélectionné dans le groupe consistant en une démence, un trouble cognitif non démentiel et des troubles de l'apprentissage ou de la mémoire.
